# EUROPÄISCHE PATENTSCHRIFT

(11) **EP 2 513 038 B1**
(45) Veröffentlichungstag und Bekanntmachung des Hinweises auf die Patenterteilung: **25.02.2015**
(21) Anmeldenummer: 10798034.4
(22) Anmeldetag: 13.12.2010
(51) Int. Cl.: C07C 209/78, C07C 209/84, C07C 211/50

(54) **ZWEISTUFIGES VERFAHREN ZUR KOSTENGÜNSTIGEN ABSCHEIDUNG VON HOMOGENEN KATALYSATOREN BEI DER MDA-SYNTHESE**
TWO-STAGE METHOD FOR COST-EFFECTIVE SEPARATION OF HOMOGENEOUS CATALYSTS IN MDA SYNTHESIS
PROCÉDÉ EN DEUX ÉTAPES VISANT À LA SÉPARATION PEU ONÉREUSE DE CATALYSEURS HOMOGÈNES LORS DE LA SYNTHÈSE DE LA MDA

(30) Priorität: 18.12.2009 EP 09179900
(43) Veröffentlichungstag der Anmeldung: 24.10.2012
(73) Patentinhaber: BASF SE, 67056 Ludwigshafen (DE)
(72) Erfinder: SIEGERT, Markus, 69126 Heidelberg (DE); MATTKE, Torsten, 67251 Freinsheim (DE); STEINMETZ, Tilmann, 67269 Grünstadt (DE); PALLASCH, Hans-Jürgen, 67169 Kallstadt (DE); NEVEJANS, Filip, 9170 St. Gillis-Waas (BE)
(86) Internationale Anmeldenummer: PCT/EP2010/069526
(87) Internationale Veröffentlichungsnummer: WO 2011/080059

(56) Entgegenhaltungen:
- WO-A1-2008/083997

## Beschreibung

Die vorliegende Erfindung betrifft ein Verfahren zur Herstellung von Diphenylmethandiamin aus Anilin mit Formaldehyd in Gegenwart einer Säure, wobei die Säure in zwei Stufen zunächst mit Ammoniak und in der zweiten Stufe mit wässriger Alkalihydroxidlösung neutralisiert wird. Der Ammoniak wird aus der anfallenden wässrigen Phase durch Behandlung mit Oxiden und/oder Hydroxiden von Erdalkalimetallen zurückgewonnen und steht erneut für die Neutralisation zur Verfügung.

Die Herstellung von Diphenylmethandiamin (MDA) durch Umsetzung von Anilin mit Formaldehyd in Anwesenheit einer Säure ist bekannt und vielfach beschrieben. In der Praxis fällt das so hergestellte Diphenylmethandiamin stets im Gemisch mit höherkondensierten Polyphenylenpolymethylenpolyaminen an. Im Folgenden wird unter "MDA" das Gemisch aus zweikernigem Diphenylmethandiamin und höherkondensierten Poly-phenylenpolymethylenpolyaminen verstanden.

Das MDA wird in der Technik zumeist durch Umsetzung mit Phosgen zu Diphenylmethandiisocyanat (MDI) umgesetzt, das ein häufig verwendetes Grundprodukt zur Polyurethanherstellung ist. Für bestimmte Einsatzgebiete, beispielsweise als Vernetzer in Kunststoffen oder Lacken, kann auch reines 2-Kern-MDA eingesetzt werden.

In der Technik erfolgt die Herstellung des MDA, wie beschrieben, durch Umsetzung von Anilin mit Formaldehyd in Anwesenheit einer Säure. Als Säure wird üblicherweise Salzsäure eingesetzt. Derartige Verfahren sind allgemein bekannt und beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059, beschrieben. Durch die Variation des Verhältnisses von Säure zu Anilin und von Formaldehyd zu Anilin kann der Anteil des 2-Kern-Produkts im MDA je nach Wunsch eingestellt werden.

Ein Problem bei der Herstellung von MDA ist die Neutralisation der als Katalysator eingesetzten Säure.

Aus EP 1 288 190 A2 ist bekannt, das bei der Umsetzung von Anilin mit Formaldehyd in Gegenwart einer Säure erhaltene Reaktionsgemisch mit einer Base, insbesondere NaOH zu neutralisieren.

EP 1 344 766 A2 offenbart eine zweistufige Behandlung der erhaltenen Reaktionsmischung, bei der im ersten Schritt die Reaktionsmischung mit einer Base neutralisiert wird und im zweiten Schritt die abgetrennte organische, das MDA enthaltende Phase mit einer Base behandelt wird. Als Basen werden Erdalkali- und Alkalihydroxide, insbesondere Natriumhydroxid verwendet.

WO 2008/083997 A1 beschreibt ein Verfahren zur Herstellung von MDA, bei dem die als Katalysator eingesetzte Säure durch Zugabe von Ammoniak anstelle von Alkali/Erdalkalioxiden bzw. -hydroxiden neutralisiert wird. Das dabei gebildete Ammoniumsalz wird durch Umsetzen mit einem Oxid oder Hydroxid eines Erdalkalimetalls wieder zurückgewonnen.

Bei der Neutralisation der im Reaktionsaustrag der MDA-Herstellung enthaltenen Säure mit wässriger Natriumhydroxidlösung werden zwei Phasen erhalten, eine wässrige und eine organische Phase. Die wässrige Phase enthält das durch die Neutralisation der Säure entstandene Salz, die organische Phase enthält die Reaktionsprodukte sowie nicht umgesetzte organische Ausgangsverbindungen. Je nach Mengenverhältnis von eingesetztem Anilin zu Formaldehyd sowie des Mengenverhältnisses von eingesetzter Säure zu Anilin unterscheiden sich die Dichten der wässrigen und der organischen Phase. Dabei kann die wässrige Phase leichter oder schwerer als die organische Phase sein, die Dichten der beiden Phasen können auch gleich sein. Unterschiedliche Mengenverhältnisse der Ausgangsverbindungen und der als Katalysator eingesetzten Säure sind üblicherweise notwendig, um MDA mit unterschiedlichen Eigenschaften wie beispielsweise Zusammensetzung, Dimeren- und Oligomerengehalt etc. zu erhalten. Für eine technische Anwendung sollte die Phasentrennung schnell und nahezu vollständig ablaufen, dies setzt voraus, dass die beiden Phasen einen ausreichend großen Dichteunterschied aufweisen. Um für einen ausreichend hohen Dichteunterschied zwischen den beiden Phasen zu sorgen, müssen häufig technische Maßnahmen ergriffen werden, beispielsweise Verdünnen einer der Phasen oder Aufkonzentrieren durch Verdampfen von Komponenten (Anilin/Wasser). Dies bedeutet einen höheren technischen Aufwand.

Weiterhin ist bei einer einmal eingerichteten Anlage durch die Verrohrung vorgegeben, welche Phase bei der Abtrennung einem bestimmten Weiterverarbeitungsschritt zugeführt wird. Eine durch veränderte Mengenverhältnisse der Edukte bzw. der eingesetzten Säure induzierte Phasenumkehr würde daher einen Umbau oder einen von vornherein komplexeren Aufbau der Anlage wie beispielsweise Verlagerung der Trennschicht und zusätzliche Ventile erfordern.

Ein weiteres Problem bei der MDA-Herstellung ist das Auftreten unerwünschter Nebenprodukte, die bei der Weiterverarbeitung des MDAs zu MDI zu unerwünschten Einfärbungen führen können. Bei der Synthese von MDA aus Formaldehyd und Anilin werden auch N-Aminobenzylaniline gebildet. Diese Verbindungen lagern im Sauren und bei erhöhter Temperatur zu MDA um. Erhöhte Werte an N-Aminobenzylanilinen führen bei der Phosgenierung des MDAs zu MDI zur Bildung von Carbamoylchloriden, die sich bei der Phosgenierung nicht in Isocyanate spalten, so dass das Endprodukt chlorhaltig ist. Weiterhin können aus den N-Aminobenzylamilinen durch weitere Kondensation mit Formaldehyd Dihydrochinazoline gebildet werden, von denen bekannt ist, dass sie die Farbwerte des aus dem MDA hergestellten MDIs negativ beeinflussen.

Aufgabe der vorliegenden Erfindung war die Bereitstellung eines Verfahrens zur Herstellung von MDA, bei dem die beiden Phasen, die bei der Neutralisation der im Reaktionsaustrag aus der MDA-Herstellung enthaltenen Säure entstehen, unabhängig von den eingesetzten Mengenverhältnissen von Anilin, Formaldehyd und Säure einen ausreichenden Dichteunterschied aufweisen und stets die selbe Phase die höhere bzw. niedrigere Dichte aufweist, so dass auf technische Maßnahmen zum Einstellen eines ausreichend großen Dichteunterschiedes verzichtet werden kann und eine bestehende Anlage ohne Umbauten oder zusätzliche Einbauten unabhängig von den eingesetzten Mengenverhältnissen der Edukte und der Säure betrieben werden kann.

Die Aufgabe wurde gelöst durch das nachfolgende Verfahren zur Herstellung von Diphenylmethandiamin und höher kondensierter Polyphenylenpolymethylenpolyaminen, umfassend die Schritte
a) Umsetzung von Anilin mit Formaldehyd in Anwesenheit einer Säure,
b) Neutralisation des überwiegenden Teils der Säure mit Ammoniak und/oder wässriger Ammoniaklösung,
c) Trennung des Reaktionsgemisches aus Schritt b) in eine wässrige und eine organische Phase,
d) Neutralisation des übrigen in der organischen Phase enthaltenen Teils der Säure mit wässriger Alkalihydroxidlösung,
e) Trennung des Reaktionsgemisches aus Schritt d) in eine wässrige und eine organische Phase,
f) Behandlung der in Schritt c) erhaltenen wässrigen Phase oder gegebenenfalls der vereinigten wässrigen Phasen aus den Schritten c) und e) mit mindestens einem Oxid oder Hydroxid eines Erdalkalimetalls,
g) Abtrennung des in Schritt f) erhaltenen Ammoniaks.

Bei der Verwendung von Ammoniak als Neutralisationsmittel ist die organische Phase schwerer als die wässrige Phase, unabhängig von den eingesetzten Mengenverhältnissen Anilin : Formaldehyd und Säure : Anilin. Technische Maßnahmen, die üblicherweise zur Erhöhung der Dichtedifferenz zwischen der wässrigen und der organischen Phase bei der Neutralisation der als Katalysator eingesetzten Säure erforderlich sind, können eingespart werden. Da im ersten Neutralisationsschritt (Schritt b)) der überwiegende Teil der Säure aus der organischen Phase bereits entfernt wurde, treten beim zweiten Neutralisationsschritt mit wässriger Alkalihydroxidlösung die vorstehend beschriebenen Probleme der variierenden Dichteunterschiede nicht mehr auf, da die Menge des bei der Neutralisation gebildeten Natriumchlorids verhältnismäßig klein ist. Die Dichte der eingesetzten wässrigen Natriumhydroxidlösung ändert sich durch die Aufnahme des Natriumchlorids nur wenig und bleibt deutlich größer als die Dichte der organischen Phase. Durch die Verwendung von wässriger Natriumhydroxidlösung in der zweiten Stufe der Neutralisation wird sichergestellt, dass die gesamte, als Katalysator eingesetzte Säure neutralisiert wird. Der zweite Neutralisationsschritt mit wässriger Alkalihydroxidlösung hat weiterhin den Vorteil, in der organischen Phase verbliebenen Ammoniak sowie darin verbliebene Ammoniumsalze als Ammoniak durch Umsalzung zu entfernen.

Überaschenderweise zeigt MDA, das aus nach dem erfindungsgemäßen Verfahren hergestellt wird, bessere Farbwerte als MDA, bei dessen Herstellung ebenfalls einstufig mit Ammoniak, einstufig mit Natronlauge oder zweistufig mit Natronlauge in beiden Stufen neutralisiert wird.

Das erfindungsgemäße Verfahren zeichnet sich weiterhin durch den kostensparenden Ersatz eines großen Teils des üblicherweise als Neutralisationsmittels verwendeten Natriumhydroxids durch die deutlich kostengünstigeren Erdalkalioxide aus. Dieser teilweise Ersatz erfolgt unter der Vermittlung durch Ammoniak, das als primäres Neutralisationsmittel eingesetzt und durch Umsetzung beispielsweise mit CaO zurückgewonnen wird und erneut eingesetzt werden kann.

Die Herstellung des MDA in Schritt a) erfolgt, wie vorstehend beschrieben, durch Umsetzung von Anilin mit Formaldehyd in Gegenwart von Säuren als Katalysatoren. Derartige Verfahren sind allgemein bekannt und beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059, beschrieben. Vorzugsweise wird als Säure in Schritt a) eine Mineralsäure, insbesondere Salzsäure, eingesetzt.

An Stelle von oder im Gemisch mit Formaldehyd kann auch mindestens eine Formaldehyd abspaltende Verbindung eingesetzt werden. Insbesondere wird der Formaldehyd als wässrige Formalinlösung, alkoholische Formalinlösung, Methylhalbacetal, Methylenimin eines primären Amins oder N,N'-Methylendiamin eines primären oder sekundären Amins sowie Paraformaldehyd eingesetzt.

Das erfindungsgemäße Verfahren kann kontinuierlich, halbkontinuierlich oder batchweise, vorzugsweise kontinuierlich oder halbkontinuierlich, durchgeführt werden.

Bei der kontinuierlichen Fahrweise werden die Reaktanden in dem gewünschten Verhältnis zueinander in einen Reaktor eindosiert und diesem Reaktor eine dem Zustrom gleiche Menge an Reaktionsprodukt entnommen. Als Reaktoren kommen beispielsweise Rohrreaktoren zum Einsatz. Bei der batchweisen oder halbkontinuierlichen Fahrweise werden die Reaktanden in einen vorzugsweise mit einem Rührer und/oder einem Umpumpkreis versehenen Batchreaktor dosiert, aus dem das ausreagierte Reaktionsprodukt entnommen und der Aufarbeitung zugeführt wird.

Das erfindungsgemäße Verfahren wird üblicherweise bei einem molaren Verhältnis von Anilin zu Formaldehyd von 20 bis 1,5, bevorzugt 10 bis 1,6, besonders bevorzugt von 2,5 bis 1,8 durchgeführt. Das molare Verhältnis von Säure zu Anilin liegt bevorzugt bei 0,04 bis 0,5, besonders bevorzugt bei 0,04 bis 0,25. Bei diesen Verhältnissen kommt es zu einer verstärkten Bildung der jeweiligen Zweikernprodukte in der Reaktionsmischung.

Als Säure werden bevorzugt Mineralsäuren wie Salzsäure, Schwefelsäure und Phosphorsäure und ganz besonders bevorzugt Salzsäure eingesetzt.

Die Reaktion wird vorzugsweise bei einer Temperatur im Bereich zwischen 0 und 200 °C, vorzugsweise zwischen 20 und 150 °C und insbesondere zwischen 40 und 120 °C durchgeführt. Es hat sich gezeigt, dass mit der Erhöhung der Temperatur der Anteil der 2,2'- und 2,4'-Isomeren im Reaktionsprodukt ansteigt.

Der Druck bei der Umsetzung beträgt 0,1 - 50, bevorzugt 1 - 10 bar absolut.

Bei der batchweisen und halbkontinuierlichen Durchführung der Reaktion kann nach der vollständigen Dosierung der Einsatzstoffe das Reaktionsgemisch einer sogenannten Alterung unterzogen werden. Dazu wird das Reaktionsgemisch im Reaktor belassen oder in einen anderen, vorzugsweise gerührten Reaktor überführt. Dabei liegt die Temperatur des Reaktionsgemisches vorzugsweise über 75 °C, insbesondere in einem Bereich zwischen 110 und 150 °C.

An die Herstellung in Schritt a) schließt sich die Neutralisation des überwiegenden Teils der Säure in Schritt b) des Reaktionsgemischs an. Dazu wird der Reaktionsmischung Ammoniak zugesetzt. Dieses kann der Reaktionsmischung gasförmig und gegebenenfalls mit Wasser gesättigt, als wässrige Ammoniaklösung oder als Mischung beider Phasen zugeführt werden. Vorzugsweise wird der Ammoniak als wässrige Lösung zugegeben. Die Konzentration des Ammoniaks beträgt dabei bevorzugt 14 bis 60 Gew.-%, gemessen bei 3 bar und Raumtemperatur, besonders bevorzugt 25 Gew-%. Bevorzugt werden mindestens 60%, besonders bevorzugt mindestens 70% und insbesondere mindestens 90% der in der Reaktionsmischung vorhandenen Säure durch den zugegebenen Ammoniak neutralisiert.

Erfindungsgemäß wird der Ammoniak in einer 1-bis 2-fachen stöchiometrischen Menge, bezogen auf die als Katalysator eingesetzte Säure zugegeben, vorzugsweise in einer 1-bis 1,7-fachen stöchiometrischen Menge. Der Ammoniak und/oder die wässrige Ammoniaklösung kann in einem Schritt oder in mehreren aufeinander folgenden Schritten zugegeben werden. Falls der Ammoniak und/oder die wässrige Ammoniaklösung in mehreren Schritten zugegeben wird, können auch die dabei entstehenden beiden Phasen vor der nächsten Zugabe getrennt werden, wobei jeweils die organische Phase erneut mit Ammoniak und/oder wässriger Ammoniaklösung versetzt wird. Ammoniak und wässrige Ammoniaklösung können dabei auch jeweils abwechselnd in den einzelnen Schritten eingesetzt werden.

Die Zusammenführung des Ammoniaks mit der Reaktionsmischung erfolgt üblicherweise in einem geeigneten Mischapparat wie einem Rührkessel, einem Rohr, das gegebenenfalls mit statischen Mischelementen versehen ist, oder anderen Apparaten. Die Zugabe basischen Ammoniaks bewirkt eine Neutralisation des überwiegenden Teils der in der Reaktionsmischung enthaltenen Säure und dadurch die Bildung zweier nichtmischbarer Phasen, einer leichteren wässrigen und einer schwereren organischen Phase. Die Neutralisation erfolgt bei einer mittleren Temperatur von 40 bis 120 °C und einem Druck von 1 bis 10 bar absolut.

Das Gemisch aus Schritt b) liegt, wie beschrieben, in einer organischen und einer wässrigen Phase vor. Die beiden Phasen werden in Schritt c) beispielsweise durch Dekantieren voneinander getrennt. Im Vergleich zur Neutralisation mit wässriger Natronlauge hat sich überraschenderweise gezeigt, dass bei Verwendung von wässriger Ammoniaklösung als Neutralisationsmittel die Phasengrenze besser ausgebildet ist. Dies bedeutet eine Entlastung insbesondere der nachfolgenden Wäsche mit wässriger Waschphase.

Nach Abtrennung der wässrigen Phase, die das Ammoniumsalz der als Katalysator eingesetzten Säure enthält, wird der in der organischen Phase verbliebene übrige Teil der Säure in Schritt d) mit wässriger Alkalihydroxidlösung neutralisiert. Dafür können z.B. Natriumhydroxid und Kaliumhydroxid eingesetzt werden, vorzugsweise wird Natriumhydroxid verwendet.

Die wässrige Natriumhydroxidlösung wird erfindungsgemäß bis maximal einem stöchiometrischen Überschuss von 2, bezogen auf die ursprünglich als Katalysator im Reaktionsgemisch eingesetzten Säure zugegeben, bevorzugt bis maximal einem stöchiometrischen Verhältnis von 1, besonders bevorzugt von maximal 0,5.

In einer bevorzugten Ausführungsform der Erfindung wird in Schritt d) der pH-Wert der organischen Phase auf > 8,5, bevorzugt auf > 9, besonders bevorzugt auf einen Wert > 9,5 und ganz besonders bevorzugt > 11 eingestellt. So kann sichergestellt werden, dass auch in den üblicherweise sehr großvolumigen Produktionsanlagen für MDA die gesamte, in der MDA-haltigen Phase vorhandene Säure neutralisiert wird.

Die Zusammenführung der wässrigen Alkalihydroxidlösung mit der Reaktionsmischung erfolgt üblicherweise in einem geeigneten Mischapparat wie einem Rührkessel, einem Rohr, das gegebenenfalls mit statischen Mischelementen versehen ist, oder anderen Apparaten. Die Zugabe der wässrigen Alkalihydroxidlösung bewirkt eine Neutralisation der Reaktionsmischung und dadurch die Bildung zweier nichtmischbarer Phasen, der wässrigen und der organischen Phase. Die Neutralisation erfolgt bei einer mittleren Temperatur von 40 bis 120 °C und einem Druck von 1 bis 10 bar absolut. Bei der Neutralisation mit wässriger Alkalihydroxidlösung in Schritt d) entstehen eine leichtere organische und eine schwerere wässrige Phase. In den Schritten b) und d) wird das jeweilige Neutralisationsmittel bevorzugt in Gegenstrom- oder Gleichstromfahrweise zugegeben.

Das aus Schritt d) stammende, als zweiphasige Mischung aus wässriger und organischer Phase vorliegende Gemisch wird in Schritt e) getrennt, beispielsweise durch Dekantieren. Die wässrige Phase aus Schritt e) kann mit der aus Schritt c) stammenden wässrigen Phase vereinigt werden und gemeinsam aufgearbeitet werden. Die beiden wässrigen Phasen können jedoch auch getrennt voneinander aufgearbeitet werden.

Gemäß einer bevorzugten Ausführungsform der Erfindung wird die in Schritt e) abgetrennte wässrige Phase zur Neutralisation in Schritt d) und/oder Schritt b) zurückgeführt. Dies ist vorteilhaft, wenn die wässrige Phase noch nicht verbrauchtes Alkalihydroxid enthält. Dies ist in der Regel der Fall, wenn die zweite Neutralisationsstufe mit einem Überschuss an Hydroxid, bezogen auf die Menge nicht in der ersten Neutralisationsstufe neutralisierte Säure, durchgeführt wird.

Die aus Schritt c) erhaltene wässrige Phase, die im wesentlichen aus Wasser, dem darin gelösten Ammoniumsalz der als Katalysator eingesetzten Säure sowie Spuren der Einsatzstoffe Anilin und Formaldehyd und auch des Endprodukts MDA besteht, wird, gegebenenfalls nach Vereinigung mit der aus Schritt e) stammenden wässrigen Phase, in Schritt f) mit mindestens einem Oxid und/oder Hydroxid eines Erdalkalimetalls behandelt. Vorzug hat Calciumoxid und/oder Calciumhydroxid, da diese gut verfügbar sind und die entstehenden Abprodukte unproblematisch zu handhaben und zu entsorgen sind. Das Calciumoxid/-hydroxid kann beispielsweise in Form von Kalkmilch bzw. Löschkalk zum Einsatz kommen. Dabei wird das Ammoniumsalz unter Bildung von Ammoniak zersetzt. Dieser Verfahrensschritt ist als Teilschritt aus dem SOLVAY-Prozess zur Herstellung von Soda bekannt. Die Abtrennung des Ammoniaks erfolgt bevorzugt destillativ oder durch Strippen mit Wasserdampf oder einem Inertgas.

Die ammoniakreiche Gasphase wird gegebenenfalls in weiteren Schritten, wie Trocknung durch Adsorption, Destillation oder Auskondensieren von Wasserdampf aufkonzentriert und gereinigt, besonders bevorzugt ist die Aufarbeitung durch Destillation. In einer bevorzugten Ausführungsform der Erfindung wird der aufgearbeitete Ammoniak wieder der Neutralisation in Schritt b) zugeführt.

In einer besonderen Ausführungsform wird das Wasserdampf und Ammoniak enthaltende Gas über Calciumoxid, auch als Branntkalk bezeichnet, geführt. Dabei wird einerseits das Gas getrocknet und zum zweiten der Branntkalk in Calciumhydroxid, sogenannten Löschkalk, überführt, der wiederum der Zersetzung des Ammoniumsalzes in Schritt f) zugeführt wird.

Die nach der Entfernung des Ammoniaks verbleibende ammoniakarme flüssige Phase kann, gegebenenfalls nach weiterer Aufkonzentration bzw. Aufreinigung, als Abwasser entsorgt werden.

Die in Schritt e) erhaltene organische Phase, die überwiegend aus MDA mit Resten von Wasser, Ammoniak, Alkalihydroxid und den Einsatzprodukten für die Herstellung des MDA besteht, wird ebenfalls aufgearbeitet. Dies erfolgt beispielsweise durch ein- oder mehrmaliges Waschen mit Wasser und/oder bevorzugt durch mehrfache Destillation. Bevorzugt wird nach Schritt e) Wasser und nicht umgesetztes Anilin aus der organischen Phase abgetrennt, beispielsweise durch Destillation.

Das nach dem erfindungsgemäßen Verfahren hergestellte MDA wird üblicherweise mit Phosgen zu MDI umgesetzt. Derartige Verfahren sind allgemein bekannt und vielfach beschrieben, beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059 oder WO 99/54289.

Dazu wird üblicherweise das MDA und gegebenenfalls das Phosgen in einem inerten Lösungsmittel gelöst und zur Reaktion gebracht. Als Lösungsmittel werden vorzugsweise inerte organische, insbesondere aromatische Lösungsmittel, wie Toluol oder halogenierte aromatische Verbindungen, wie Monochlorbenzol, eingesetzt.

Die Phasengewinnung kann in üblichen Reaktoren, beispielsweise Rührkesseln, Rührkesselkaskaden, Kolonnen und/oder Rohrreaktoren bei bekannten Temperaturen von z. B. 50 bis 150 °C, bevorzugt 70 bis 120 °C, besonders bevorzugt 70 bis 100 °C und einem Druck von 0,5 bis 10 bar durchgeführt werden.

Beispielsweise kann die Phosgenierung als zweistufige Umsetzung in Gegenwart mindestens eines inerten organischen Lösungsmittels durchgeführt werden, wobei die erste Stufe der Phosgenierung in einem statischen Mischer und die zweite Stufe der Phosgenierung in einem Verweilzeitapparat durchgeführt werden.

Das durch die Phosgenierung hergestellte Roh-MDI kann durch übliche Verfahren, beispielsweise Destillation, gereinigt werden. Bevorzugt kann in einem ersten Reinigungsvorgang Phosgen und gegebenenfalls Lösungsmittel, bevorzugt weitgehend, besonders bevorzugt vollständig aus dem Reaktionsgemisch der Phosgenierung, d. h. dem Roh-MDI entfernt werden.

Bevorzugt kann anschließend gewünschtes monomeres MDI, beispielsweise 2,2'-, 2,4'- und/oder 4,4'-MDI und/oder Gemische enthaltend mindestens zwei diese Isomere, durch ein geeignetes Verfahren, bevorzugt durch Destillation, beispielsweise bei Drücken von 2 bis 50 mbar, bevorzugt 2 bis 20 mbar, und Temperaturen von 150 bis 250°C, bevorzugt 180 bis 230°C, und/oder bevorzugt durch Kristallisation, beispielsweise fraktionierte Kristallisation, abgetrennt werden.

In einer besonderen Ausführungsform des Verfahrens zur Herstellung von MDI kann aus dem Roh-MDA das Zwei-Kern-Produkt abgetrennt und mittels Gasphasenphosgenierung, wie beispielsweise EP 570 799 beschrieben, zu Zwei-Kern-MDI umgesetzt werden.

Das so hergestellte MDI kann insbesondere mit Verbindungen mit mindestens zwei aktiven Wasserstoffatomen zu Polyurethanen umgesetzt werden.

Das erfindungsgemäße Verfahren erlaubt eine kostengünstige und betriebssichere Aufarbeitung von MDA. Es kommt zu keiner Schädigung des MDA. Das eingesetzte Ammoniak kann vollständig vom Reaktionsprodukt abgetrennt werden. Durch die Kreislaufführung des Ammoniaks werden Produktverluste vermieden. Die entstehenden wässrigen Salzlösungen können problemlos entsorgt werden. Das aus dem erfindungsgemäßen Verfahren hergestellte MDA durch Phosgenierung hergestellte MDI weist verbesserte Farbwerte auf.

Die Erfindung wird in den nachfolgenden Beispielen näher erläutert.

### Beispiele:

### Beispiel 1: Dichteunterschiede

Die Dichteunterschiede für die organische und die wässrige Phase nach Neutralisation mit wässriger Natriumhydroxidlösung bzw. mit wässriger Ammoniaklösung für den MDI-Reaktionsaustrag der Umsetzung von Anilin mit Formaldehyd in Gegenwart von HCl bei unterschiedlichen Verhältnissen von Anilin zu Formaldehyd (A/F) sowie Säure zu Anilin (S/A) wurden berechnet.

**Tabelle 1**

| | wässrige NaOH-Lösung Dichteunterschied [kg/m³] | | wässrige NH₃-Lösung Dichteunterschied [kg/m³] | |
|---|---|---|---|---|
| Säure/Anilin [mol/mol] | Anilin/ Formaldehyd: 1,8 mol/mol | Anilin/ Formaldehyd: 2,4 mol/mol | Anilin/ Formaldehyd: 1,8 mol/mol | Anilin/ Formaldehyd: 2,4 mol/mol |
| 0,04 | 84,65 | 43 | 120 | 87 |
| 0,1 | 41 | -5 | 95 | 57 |
| 0,24 | -7,23 | -52 | 77 | 37 |

| | | | | |
|---|---|---|---|---|
| Dichteunterschied: Dichte (organische Phase) - Dichte (wässrige Phase) | | | | |

Ein negatives Vorzeichen bedeutet, dass eine Phasenumkehr stattgefunden hat und die wässrige Phase die schwerere Phase ist.

Der Dichteunterschied der beiden Phasen ändert sich bei Neutralisation mit wässriger Natriumhydroxidlösung mit steigendem S/A-Verhältnis deutlich, bei einem A/F-Verhältnis von 2,4 mol/mol findet zwischen dem S/A-Verhältnis von 0,05 und 0,1 eine Phasenumkehr statt, bei einem A/F-Verhältnis von 1,8 mol/mol wird die Dichtedifferenz zwischen wässriger und organischer Phase mit steigendem S/A-Verhältnis ebenfalls kleiner, die Phasenumkehr findet zwischen einem S/A-Verhältnis von 0,2 und 0,25 statt.

Bei der Neutralisation mit wässriger NH₃-Lösung weist der Dichteunterschied stets das gleiche Vorzeichen auf, somit tritt keine Phasenumkehr auf, auch sind die Absolutwerte groß genug, um eine schnelle Phasentrennung zu ermöglichen.

### Beispiel 2: Herstellung von MDA aus Anilin und Formaldehyd unter Katalyse mit HCl

Formaldehyd und Anilin (Formaldehyd/Anilin = 2,4 mol/mol) wurden in Gegenwart von Salzsäure (HCl/Anilin = 0,07 mol/mol) bei 50-90°C und 1-1,5 bar umgesetzt.

### Beispiel 3 (erfindungsgemäß): zweistufige Neutralisation mit NH₃ in der ersten und wässriger NaOH in der zweiten Stufe

2000 g des Reaktionsaustrags gemäß Beispiel 1 wurden mit 200 g 25%-iger wässriger Ammoniaklösung gemischt. Der pH-Wert betrug 8,9. Die wässrige Phase war leichter als die organische Phase. Die beiden Phasen wurden getrennt. Im nächsten Schritt wurde der organischen Phase ca. 77 g Natronlauge (50 Gew.-%) zugegeben. Der pH-Wert der Mischung betrug 9,7. Nach erfolgter Phasentrennung wurden die Phasen getrennt aus der Laborapparatur abgezogen. Die abgezogene wässrige Oberphase enthielt etwa 6,24 Gew.-% Chlorid-Ionen und jeweils einen Anteil von ca. 3 Gew.-% Natriumionen und 3,1 Gew.-% Ammoniumionen.

Nach der Phasentrennung wurden 956 g der organischen Phase zweistufig mit VE-Wasser (Phasenverhältnis 0,28 kg/kg) gewaschen und das Anilin und Restwasser bei 125°C und 18 mbar abdestilliert. Anschließend erfolgte die Phosgenierung der organischen Probe.

### Beispiel 4 (Vergleich): einstufige Neutralisation mit NH₃

2 kg des Reaktionsaustrags gemäß Beispiel 1 wurden mit ca. 200 g 25%-iger wässriger Ammoniaklösung gemischt. Der pH-Wert betrug 9,0. Die wässrige Phase war leichter als die organische Phase. Nach erfolgter Phasentrennung wurden die beiden Phasen getrennt aus der Laborapparatur abgezogen. Die abgezogene wässrige Oberphase enthielt etwa 7 Gew.-% Chlorid-Ionen und ca. 5 Gew.-% Ammonium-Ionen.

Nach der Phasentrennung wurden 956 g der organischen Phase zweistufig mit VE-Wasser (Phasenverhältnis 0,28 kg/kg) gewaschen und das Anilin und Restwasser bei 140°C und 17 bar abdestilliert. Anschließend erfolgte die Phosgenierung der organischen Phase.

### Beispiel 5 (Vergleich): einstufige Neutralisation mit wässriger NaOH

2,4 kg des Reaktionsaustrags gemäß Beispiel 1 wurden mit 102 g wässriger 50%-iger Natronlauge versetzt. Der pH-Wert betrug 10,8. Die wässrige Phase bildet die untere Phase. Die Phasen wurden getrennt aus der Laborapparatur abgezogen. Die abgezogene wässrige Unterphase enthielt etwa 8 Gew.-% Chlorid-Ionen.

Nach erfolgter Phasentrennung wurden 956 g der organischen Phase zweistufig mit VE-Wasser (Phasenverhältnis etwa 0,28 kg/kg) gewaschen und das Anilin und Restwasser bei 140°C und 17 mbar abdestilliert. Anschließend erfolgte die Phosgenierung der organischen Probe.

### Beispiel 6 (erfindungsgemäß): Neutralisation mit gasförmigem NH₃, wässriger NH₃-Lösung und wässriger NaOH-Lösung

1400 g des Reaktionsaustrags gemäß Beispiel 1 wurden mit 57 g gasförmigem NH₃ versetzt. Der pH-Wert betrug 7,4. Die organische Phase war schwerer als die wässrige Phase. Die abgetrennte organische Phase (916 g) wurde mit 227 g 25%-iger wässriger NH₃-Lösung versetzt, wobei sich ein pH-Wert von 9,45 bei 73°C einstellte. Dabei wurde ständiges Ausgasen beobachtet. Nach Aufheizen fiel der pH-Wert auf 8,5. Anschließend wurden 3,7 g wässrige NaOH-Lösung (30%) zugegeben. Der pH-Wert betrug danach 9,5.

### Beispiel 7: Bestimmung des Gehalts an Dihydrochinazolin

Der Gehalt an Dihydrochinazolin wurde durch gaschromatographische Analyse der aus Beispiel 3 erhaltenen gelösten Proben bestimmt. Als Lösungsmittel wurde Dimethylformamid (DMF) mit 3 mg Tetramethyl-MDA pro mL DMF eingesetzt. Die Kalibrierung erfolgte mit Lösungen von 2-Kern-Dihydrochinazolin, von 4,4'-MDA und 3-Kern-MDA. Es wurde ein Gaschromatograph (HP 5890) mit Flammenionisationsdetektor verwendet. Als Trägergas wurde H₂ verwendet. Die Ergebnisse der Analyse sind in Tabelle 2 dargestellt. Ein Wert ≤ 10 bedeutet, dass der Gehalt unterhalb der Nachweisgrenze lag.

**Tabelle 2**

| Gehalt [ppm] | MDA aus Bsp. 3, (erfindungsgemäß) | MDA aus Bsp. 4, (Vergleich) | MDA aus Bsp. 5, (Vergleich) |
|---|---|---|---|
| 2 Kern-Dihydrochinazolin | ≤ 10 | ≤ 10 | ≤ 10 |
| 3 Kern-Dihydrochinazolin | ≤ 10 | ≤ 10 | 75 |
| 4 Kern-Dihydrochinazolin | 11 | 19 | 90 |

Das nach dem erfindungsgemäßen Verfahren hergestellte MDI weist einen niedrigeren Gesamtgehalt an den Farbwert negativ beeinflussenden Dihydrochinazolinen auf.

### Beispiel 8: Bestimmung der Farbwerte

Die Farbwerte der aus Beispiel 3 erhaltenen MDIs wurden iodometrisch bestimmt. Ein hoher Wert bedeutet starke Einfärbung, ein niedriger Wert eine schwache Einfärbung.

Die Ergebnisse sind in Tabelle 3 dargestellt.

**Tabelle 3**

| | Jod-Farbzahl |
|---|---|
| MDA aus Bsp. 3 (erfindungsgemäß) | 23,2 |
| MDA aus Bsp. 4 (Vergleich) | 23,3 |
| MDA aus Bsp. 5 (Vergleich) | 28,8 |

Das erfindungsgemäße Verfahren führt zu MDI mit niedrigeren Farbwerten.

### Beispiel 9 (erfindungsgemäß): Rückgewinnung des Ammoniaks

Zu 1000 g der aus der ersten Neutralisation des Beispiels 1 erhaltenen wässrigen Phase wurde in einer temperierten Laborapparatur 150 g Calciumhydroxid (Löschkalk) gegeben. Der dabei entstandene, Wasser enthaltende gasförmige Ammoniak wurde über einen mit Calciumoxid gefüllten Trockenturm geführt und so getrocknet. Der getrocknete Ammoniak konnte erneut zur Neutralisation eingesetzt werden. Nach vollständiger Entgasung verblieb in der Apparatur eine etwa 20%-ige wässrige Lösung von Calciumchlorid zur Entsorgung.

## Patentansprüche

1. Verfahren zur Herstellung von Diphenylmethandiamin und höherkondensierten Polyphenylenpolymethylenpolyaminen, umfassend die Schritte
a) Umsetzung von Anilin mit Formaldehyd in Anwesenheit einer Säure,
b) Neutralisation des überwiegenden Teils der Säure mit Ammoniak und/oder wässriger Ammoniaklösung,
c) Trennung des Reaktionsgemisches aus Schritt b) in eine wässrige und eine organische Phase,
d) Neutralisation des übrigen in der organischen Phase enthaltenen Teils der Säure mit wässriger Alkalihydroxidlösung,
e) Trennung des Reaktionsgemisches aus Schritt d) in eine wässrige und eine organische Phase,
f) Behandlung der in Schritt c) erhaltenen wässrigen Phase oder gegebenenfalls der vereinigten wässrigen Phasen aus den Schritten c) und e) mit mindestens einem Oxid oder Hydroxid eines Erdalkalimetalls,
g) Abtrennung des in Schritt f) erhaltenen Ammoniaks.

2. Verfahren nach Anspruch 1, **dadurch gekennzeichnet, dass** in Schritt a) als Säure eine Mineralsäure eingesetzt wird.

3. Verfahren nach Anspruch 1 oder 2, **dadurch gekennzeichnet, dass** in Schritt a) als Säure Salzsäure eingesetzt wird.

4. Verfahren nach einem der Ansprüche 1 bis 3, **dadurch gekennzeichnet, dass** in Schritt b) der Ammoniak als wässrige Lösung zugegeben wird.

5. Verfahren nach einem der Ansprüche 1 bis 4, **dadurch gekennzeichnet, dass** in den Schritten b) und d) das Neutralisationsmittel in Gegenstrom- oder Gleichstromfahrweise zugeben wird.

6. Verfahren nach einem der Ansprüche 1 bis 5, **dadurch gekennzeichnet, dass** die in Schritt e) abgetrennte wässrige Phase zur Neutralisation in Schritt d) zurückgeführt wird.

7. Verfahren nach einem der Ansprüche 1 bis 6, **dadurch gekennzeichnet, dass** in Schritt f) als Oxid oder Hydroxid eines Erdalkalimetalls Calciumoxid und/oder Calciumhydroxid eingesetzt wird.

8. Verfahren nach einem der Ansprüche 1 bis 7, **dadurch gekennzeichnet, dass** in Schritt f) als Hydroxid eines Erdalkalimetalls Calciumhydroxid eingesetzt wird.

9. Verfahren nach einem der Ansprüche 1 bis 8, **dadurch gekennzeichnet, dass** das in Schritt g) erhaltene Ammoniak in den Schritt b) zurückgeführt wird.

10. Verfahren nach einem der Ansprüche 1 bis 9, **dadurch gekennzeichnet, dass** nach Schritt e) oder g) Wasser und nicht umgesetztes Anilin aus der organischen Phase abgetrennt werden.

11. Verfahren nach einem der Ansprüche 1 bis 10, **dadurch gekennzeichnet, dass** die in Schritt e) erhaltene organische Phase gereinigt und zu Diphenylmethandiamin und höher kondensierten Polyphenylenpolymethylenpolyaminen aufgearbeitet wird.

12. Verfahren nach einem der Ansprüche 1 bis 11, **dadurch gekennzeichnet, dass** das gereinigte Diphenylmethandiamin mit Phosgen zu Diphenylmethandiisocyanat umgesetzt wird.

## Claims

1. A process for preparing diphenylmethanediamine and more highly condensed polyphenylenepolymethylenepolyamines, comprising the steps of:
a) reacting aniline with formaldehyde in the presence of an acid,
b) neutralizing the predominant part of the acid with ammonia and/or aqueous ammonia solution,
c) separating the reaction mixture from step b) into an aqueous phase and an organic phase,
d) neutralizing the other part of the acid, present in the organic phase, with aqueous alkali metal hydroxide solution,
e) separating the reaction mixture from step d) into an aqueous phase and an organic phase,
f) treating the aqueous phase obtained in step c) or optionally the combined aqueous phases from steps c) and e) with at least one oxide or hydroxide of an alkaline earth metal,
g) removing the ammonia obtained in step f).

2. The process according to claim 1, wherein a mineral acid is used as acid in step a).

3. The process according to claim 1 or 2, wherein hydrochloric acid is used as acid in step a).

4. The process according to any of claims 1 to 3, wherein the ammonia in step b) is added as an aqueous solution.

5. The process according to any of claims 1 to 4, wherein the neutralizing agent is added countercurrentwise or cocurrentwise in steps b) and d).

6. The process according to any of claims 1 to 5, wherein the aqueous phase removed in step e) is recycled to the neutralization in step d).

7. The process according to any of claims 1 to 6, wherein calcium oxide and/or calcium hydroxide is used as oxide or hydroxide of an alkaline earth metal in step f).

8. The process according to any of claims 1 to 7, wherein calcium hydroxide is used as hydroxide of an alkaline earth metal in step f).

9. The process according to any of claims 1 to 8, wherein the ammonia obtained in step g) is recycled into step b).

10. The process according to any of claims 1 to 9, wherein water and unreacted aniline are removed from the organic phase after step e) or g).

11. The process according to any of claims 1 to 10, wherein the organic phase obtained in step e) is purified and is worked up to diphenylmethanediamine and more highly condensed polyphenylenepolymethylenepolyamines.

12. The process according to any of claims 1 to 11, wherein the purified diphenylmethanediamine is reacted with phosgene to give diphenylmethane diisocyanate.

## Revendications

1. Procédé pour la préparation de diphénylméthanediamine et de polyphénylènepolyméthylènepolyamines plus hautement condensées comprenant les étapes de
a) transformation d'aniline avec du formaldéhyde en présence d'un acide,
b) neutralisation de la partie principale de l'acide avec de l'ammoniac et/ou une solution aqueuse d'ammoniac
c) séparation du mélange réactionnel de l'étape b) en une phase aqueuse et une phase organique,
d) neutralisation de la partie résiduelle de l'acide contenue dans la phase organique avec une solution aqueuse d'hydroxyde de métal alcalin
e) séparation du mélange réactionnel de l'étape d) en une phase aqueuse et une phase organique,
f) traitement de la phase aqueuse obtenue dans l'étape c) ou le cas échéant des phases aqueuses rassemblées des étapes c) et e) avec au moins un oxyde ou un hydroxyde d'un métal alcalino-terreux,
g) séparation de l'ammoniaque obtenue dans l'étape f).

2. Procédé selon la revendication 1, **caractérisé en ce qu'**on utilise dans l'étape a), comme acide, un acide minéral.

3. Procédé selon la revendication 1 ou 2, **caractérisé en ce qu'**on utilise dans l'étape a), comme acide, de l'acide chlorhydrique.

4. Procédé selon l'une quelconque des revendications 1 à 3, **caractérisé en ce que**, dans l'étape b), l'ammoniac est ajouté sous forme de solution aqueuse.

5. Procédé selon l'une quelconque des revendications 1 à 4, **caractérisé en ce que**, dans les étapes b) et d), l'agent de neutralisation est ajouté dans un mode opératoire à contre-courant ou à flux parallèles.

6. Procédé selon l'une quelconque des revendications 1 à 5, **caractérisé en ce que** la phase aqueuse séparée dans l'étape e) est recyclée dans l'étape d) pour la neutralisation.

7. Procédé selon l'une quelconque des revendications 1 à 6, **caractérisé en ce qu'**on utilise, dans l'étape f), comme oxyde ou hydroxyde d'un métal alcalino-terreux, de l'oxyde de calcium et/ou de l'hydroxyde de calcium.

8. Procédé selon l'une quelconque des revendications 1 à 7, **caractérisé en ce qu'**on utilise, dans l'étape f), comme hydroxyde d'un métal alcalino-terreux, de l'hydroxyde de calcium.

9. Procédé selon l'une quelconque des revendications 1 à 8, **caractérisé en ce que** l'ammoniaque obtenue dans l'étape g) est recyclée dans l'étape b).

10. Procédé selon l'une quelconque des revendications 1 à 9, **caractérisé en ce que** l'eau et l'aniline non transformée sont séparées de la phase organique après l'étape e) ou g).

11. Procédé selon l'une quelconque des revendications 1 à 10, **caractérisé en ce que** la phase organique obtenue dans l'étape e) est purifiée et transformée en diphénylméthanediamine et en polyphénylènepolyméthylènepolyamines plus hautement condensées.

12. Procédé selon l'une quelconque des revendications 1 à 11, **caractérisé en ce que** la diphénylméthanediamine purifiée est transformée avec du phosgène en diphénylméthanediisocyanate.
